# EUROPEAN PATENT APPLICATION

(11) **EP 4 483 969 A1**
(43) Date of publication of application: **01.01.2025**
(21) Application number: 22717215.2
(22) Date of filing: 25.02.2022
(51) Int. Cl.: A62B 18/04

(54) **AIR FILTRATION DEVICE FOR PROTECTION**

(71) Applicant: Werlax Invest, S.L., 17003 Girona (ES)
(72) Inventor: FERRER SOLER, Julia, Figueres, 17600 Girona (ES); SABRIA MESTRAS, Silvia, Sant Miquel de Fluvià, Girona 17475 (ES)
(74) Representative: ZBM Patents - Zea, Barlocci & Markvardsen
(86) International application number: PCT/ES2022/070104
(87) International publication number: WO 2023/161536

(57) **Abstract**

The air filtration device for protection (1) comprises: a securing structure (3) for securing to a user's head; a visor (2) associated with the securing structure (3); a hood (4) fixed to the securing structure, defining a closed chamber (7); a rear air inlet (5) comprising an inlet filter (5.1); an air outlet (6); and a fan (8) for generating an air flow from the air inlet (5.1) through an upper air duct (9) and through a front portion of the closed chamber (7) to the air outlet. The air outlet (6) is arranged on one side and comprises an outlet filter (6.1), a side frame (6.2) and a side cover (6.3). The outlet filter (6.1) is fixed between the side frame (6.2) and the side cover (6.3).

## Description

### Technical sector

The present invention refers to an air filtration device for protection, of the type called Personal Protection Equipment (PPE).

### State of the art

Nowadays, the use of Personal Protective Equipment (PPE) by workers in different sectors is common with the aim of obtaining protection against one or more risks that may threaten their safety or health.

One of these sectors is health, in which workers (doctors, nurses, assistants, etc.) are in direct contact with sick patients, capable of transmitting their disease through infectious agents (airborne, liquids, etc.) to healthcare workers or other patients. Likewise, there is the possibility that the health worker himself may contract a disease (for example, in the family environment) and infect the patients.

To avoid these risks, one of the PPE most used by health workers are masks (for example, those known as surgical), which consist of pieces of cloth or paper made of insulating material that are placed over the nose and mouth. and is secured with a rubber band or headband in order to prevent the transfer of infectious agents from the patient to the user or vice versa.

These masks have the great drawback that they do not completely isolate the user, running the risk of getting sick from contact with infectious agents (for example, through the eyes, skin, etc.), as well as the professional's own infectious agents (expired air, sweat, etc.) may endanger the patient's health.

Additionally, it is common to use other PPE, commonly called full face masks, which cover a large part of the user's face. These comprehensive masks They comprise a transparent visor that is attached to the head by means of straps, covering the user's face and isolating it from the outside. Said full-face masks usually include, in their lower part, an opening for the entry and exit of air which, in turn, includes an interchangeable filter for filtering the air that passes through said opening.

The main drawback of this type of full-face masks is that it is very common for the visor to fog up due to condensation of the air exhaled by the user, making it difficult to perform their tasks. This fogging of the visor can lead to very dangerous situations, such as if it occurs in the middle of an operation, where any error can endanger the patient's life.

Another drawback of these full face masks is that they do not isolate the user's entire head, leaving the user exposed to infectious agents that can enter their body through the skin or may expose patients to the user's infectious agents, putting their health at risk. health.

Additionally, the use of PPE commonly called respiratory protection hoods is known, which are comprised of a hood of insulating material and a transparent visor that define a closed chamber that surrounds the entire head of the user. These hoods usually include a hole in the back connected to a tube that leads to an external device that generates a flow of air into the hood (for example, an oxygen cylinder).

One of the drawbacks of these respiratory protection hoods is that said tube and external device make it difficult for the user to move (such as if the tube gets caught on furniture, if the external device is an oxygen bottle, etc.), which makes your job difficult.

Another drawback of this type of hood is that the air outlet is usually a hole arranged in the front part of the hood, in a section close to the user's mouth. The surface of said front part is occupied almost entirely by the visor, so there is not enough surface to house a filter with the necessary surface area for filtering the air passing through said hole. Therefore, these known hoods are not designed to prevent the user from infecting a patient. Therefore, an air filtration device is necessary that facilitates the user's freedom of movement, and isolates both the user and the patients from infectious agents.

### Object of the invention

In order to meet this objective and solve the technical problems discussed so far, in addition to providing additional advantages that can be derived later, the present invention provides an air filtration device for protection that comprises: a fastening structure configured to its attachment to the head of a user; a visor associated with the support structure; a hood associated with the fastening structure, said hood defining a closed chamber after fastening the fastening structure to the user's head; a rear air inlet comprising an inlet filter; an air outlet; and a fan configured to generate an air flow from the air inlet through an upper air duct and through a front portion of the closed chamber to the air outlet.

The air outlet is arranged on at least one side and comprises an outlet filter, a side frame and a side cover. The side cover has at least one air passage opening and a curved surface that defines an air chamber between the outlet filter and the side cover. The side cover is configured to be fixed to the side frame, so that the outlet filter is fixed between the side frame and the side cover.

Rear, top, side and front can be understood as the different positions of the device relative to the user's head when the device is in its position of use.

In this way, the combination of the visor and hood defines a closed chamber around the user's head, isolating the user's head from infectious agents coming from the outside, and vice versa. Likewise, the air flow generated by the fan supplies filtered outside air to the user, protecting them from infectious agents that could be in the air. In this way the device is not related to heavy objects that could limit the user's freedom of movement.

Furthermore, since the air flow is directed to the front of the closed chamber, the user is continuously adequately ventilated.

Thanks to the fact that the air flow flows through the front part of the closed chamber, that is, where the visor is located, the possibility of fogging of the visor due to condensation of the air exhaled by the user is reduced.

As indicated, the air outlet is arranged on at least one side of the device in a position of use, preferably around at least one ear of the user. More preferably, the air outlet is arranged on each of the two sides. In this way, the surface area available for the air filter is doubled and a better distribution of the air flow across the user's face is obtained.

In this way, it is possible to place an outlet filter with the necessary surface area, for the proper functioning of the air filtering device, at the air outlet since the side of the device comprises a large surface area not occupied by the visor.

Not only that, the arrangement of the outlet filter on the side allows for a longer useful life for the outlet filter because, due to their location and larger surface area, they accumulate a smaller amount of humidity from the air exhaled by the user, thus prolonging the life of the filter. time of use of the output filters until their saturation.

Likewise, it is contemplated that the side frame is fixed to the hood, although it is not ruled out that it is fixed to the fastening structure.

Furthermore, since the outlet filter is fixed between the side frame and the side cover, the outlet filter is easily replaceable.

Additionally, the air chamber between the outlet filter and the side cover allows most of the surface of the outlet filter to be used for filtering the air that passes through the air vent.

Preferably the side frame comprises an outer surface that is provided with ribs on which the outlet filter is arranged. This allows a space to be left between the outlet filter and the outer surface of the side frame, so that most of the outlet filter is used for filtering the air passing through the air outlet. These ribs in the side frame facilitate the condensation of humidity in the air passing through the air outlet, increasing the useful life of the outlet filter.

Preferably, the side cover comprises ribs on the curved surface where the outlet filter is arranged. Said ribs make it possible to reduce the area of the outlet filter in contact with the curved surface of the side cover. Thus, most of the outlet filter is used for filtering the air that passes through the air outlet. Likewise, these ribs on the side cover facilitate the condensation of moisture from the air passing through the air outlet, which increases the useful life of the outlet filter.

Preferably, the upper air duct comprises a plurality of subducts for a homogeneous distribution of the air flow along the visor, avoiding discomfort to the user due to the concentration of the air flow in a single section of the user's face and preventing fogging of the visor. viewfinder.

Preferably the input filter has a greater filtering capacity than the output filter. In this way, the necessary surface area of the outlet filter is reduced for the proper functioning of the air filtering device. This is because the dimension of the outlet filter is related to the flow rate of the air flow that passes through the outlet filter, that is, a greater flow rate of the air flow requires a greater surface area of the outlet filter, and therefore material of the outlet filter, that is, the greater the filter capacity of the outlet filter material, the greater the surface area of the outlet filter is required. Likewise, the air flow rate is directly related to the surface of the inlet filter, that is, the larger the surface of the inlet filter, the greater the air flow rate, the material of the inlet filter, that is, the greater the filter capacity. of the inlet filter, the lower the air flow rate, and with the power of the fan, that is, the higher the fan power, the air flow rate will be greater.

Preferably the device comprises an external power supply unit that It can be attached to the user's waist, improving the portability of the device.

Preferably, the device comprises sound amplification means that at least comprise a microphone, a headset and/or a speaker to allow communication between the user and the outside world. This ensures that the user can hear the sound from outside the closed chamber with complete clarity and/or vice versa. It is contemplated that the microphone and/or speaker are located outside the closed chamber and the earpiece and/or microphone are located inside the closed chamber. Preferably, the sound amplification means are configured to cancel the sound emitted by the air flow and/or the ambient sound from outside, for example, by canceling a specific frequency, which reduces the discomfort of the user, improving its hearing.

Preferably, the device comprises a control unit configured to regulate the speed of the fan, for example, by means of an integrated algorithm, for turning on, off, power control and/or rotation of the fan motor. This allows greater control of the air flow inside the closed chamber. To this end, it is contemplated that the control unit comprises at least one processor.

Preferably, the device comprises at least one sensor of airflow characteristics inside the closed chamber. The control unit is configured to receive information from the air flow characteristics sensor, in particular, to continuously monitor information received from the air flow characteristics sensor, and contribute to the different functions of the control unit such as, for example, fan speed adaptation.

Preferably, the air flow characteristics sensor is a flow sensor, or flow meter, that measures the flow rate at the inlet of the air flow into the closed chamber.

The use of a CO₂ concentration sensor is not ruled out so that the control unit prevents said concentration from exceeding a set CO₂ concentration value.

Likewise, it is not ruled out that the air flow characteristics sensor is a pressure, temperature, etc. sensor. Or a combination of all of them. Preferably, the control unit is configured to maintain a maximum pressure difference with respect to the external atmospheric pressure inside the closed chamber of up to 0.5 mBar. In this way, the control unit makes it possible to provide an inlet flow rate of air inside the closed chamber adequate to supply the user with sufficient breathing air for heavy duty work and, at the same time, prevent the pressure difference between the inside the closed chamber and the outside atmospheric pressure exceeds 0.5 mBar. Thanks to this feature, user inconvenience is avoided.

Preferably, the device comprises means for uniquely identifying the device by means of a unique digital label processed by the control unit. This tag will contain a series of related information, called attributes. These attributes can be modified only if a series of conditions are met. The identity of the device will, therefore, be defined by this unique tag with attributes such as the coded name of the user to whom the device is assigned, the number of times the assigned person has put on the device, the number of hours since the assigned person puts on the device until it is removed, if the CO₂ level OR air flow has exceeded a programmed limit, etc.

Preferably, the device comprises means for encrypting data processed by the control unit, in particular, by means of a cryptographic algorithm. Encryption can be carried out using "blockchain" and/or "tokenization" technology. This information that provides a digital identity to the device allows secure registration, thanks to a cryptographic algorithm, of information relevant to the device in a blockchain network, facilitating the maintenance of the device, its cleaning and a change of assignment to another user. Likewise, said relevant information may be an identity and attributes (for example, user name, battery status, hours of use, last use, last disinfection, etc.), which can be updated as the life of the device passes. useful of the device. In this way, said information is converted into non-fungal tokens ("NFTs"), which will be created in a "blockchain" and managed through a "Smart Contract". The programming of said "Smart Contract" will also allow the device to be assigned to a specific user, allowing only this user to start this device. This concept is equivalent to that of a unique electronic key that can be programmed to personalize it for a period of time, increasing the attention paid to the maintenance and security of the device by the user. The identification of various air filtration devices in a "blockchain" network will allow the programming of the "Smart Contract" to gather user information in order to have a comparison of the use of the device by user profile, its location and the need for updating in materials or electronics to generate new improvement processes in functionalities and maintenance.

Preferably, the control unit comprises wireless Internet connection means, in particular through WIFI, Bluetooth^{®} technology, Zigbee technology, Near Field Communication (NFC) technology, GSM, as well as sending and receiving point-to-point information, point such as "LoRa" and/or technology associated with the Internet of Things (oT) such as Sigfox, directly to a server or to an intermediate node, more particularly, using a frequency of the ISM (Industrial, Scientific and Medical) and a proprietary protocol.

The extraction of data between periods of use of the device will allow statistical data to be extracted to improve the device in the future and add more functionalities, so another of the attributes of the device can be the version number of the algorithm update with new functionalities.

Likewise, the object of the present invention is a cleaning system for the air filtration device that comprises means for sterilizing the device using UV-C radiation. This ensures correct disinfection of the air filtration device between uses, projecting UV-C light in a safe way that cannot be harmful to humans.

Additionally, this cleaning can be combined with charging the external power supply unit.

It is contemplated that the sterilization means may be in the form of a sheath into which the device is inserted.

With the initial data from the deployment of the use of the air filtration devices in a safe and anonymous way, an accurate prediction of potential failures can be made in order to treat them and minimize any maintenance time. The data transmitted securely in the "Blockchain" may be collected in several periods to visually represent the data of deployment, and use, allowing to have a clear visualization picture on the deployment to be able to encourage its use in certain locations.

The definition of the air filtration device as an "NFT" will allow the feedback of each user to be collected in the future for the improvement of their specific device and will also allow the exchange of some suggestions on new functionalities of use between various professionals and facilitate their acquisition and the possibilities of minimizing any negative impact on sustainability.

### Description of the figures

A non-limiting example of the present invention will now be described with reference to the accompanying drawings, in which:
Figure 1 shows a perspective view of an example of a preferred embodiment of the air filtration device according to the present invention.
Figure 2 shows an elevation view of the device of Figure 1 with some internal parts thereof visible.
Figure 3 is an exploded perspective view of the air filtration device.
Figure 4 are perspective views that show the steps to follow for the correct placement of the air filtering device.
Figure 5 is a partial perspective view of the air inlet.
Figures 6 A to 6 F are perspective views showing the steps to follow to remove the inlet filter.
Figure 7 shows a top-down view of the upper air duct where the air distribution subducts at the outlet of the air duct have been schematically illustrated. Figure 8 shows a perspective view of the explosion of the air outlet.
Figure 9 shows a perspective view of the side cover.

### Detailed description of the invention

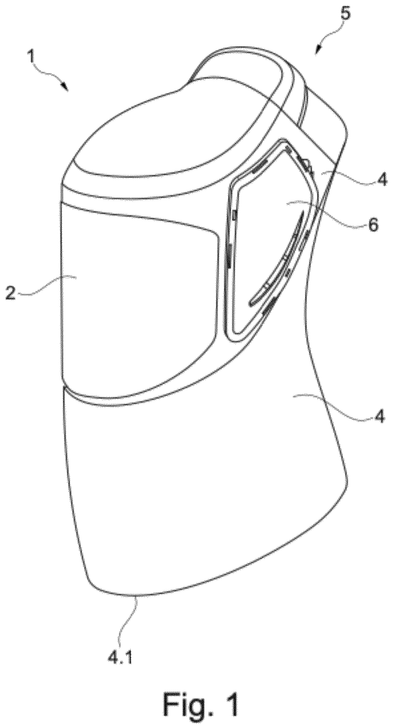

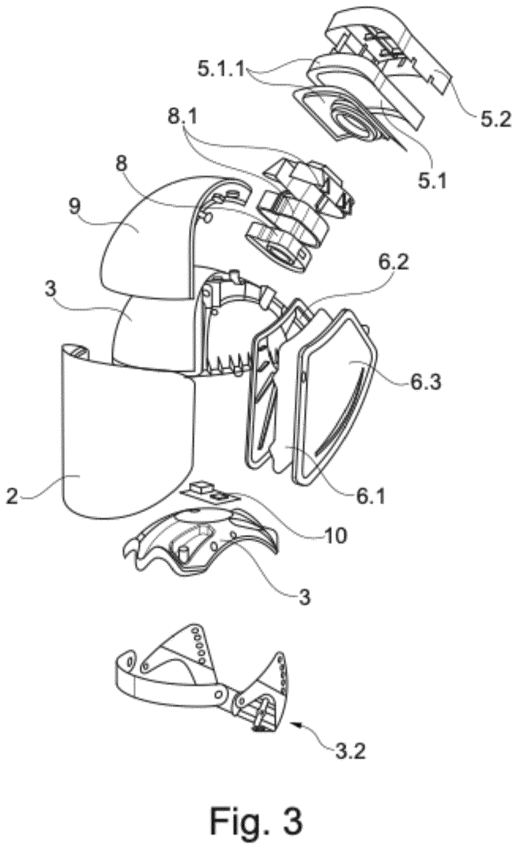

The non-limiting example illustrated in Figures 1-9 corresponds to an air filtration device (1). The air filtration device (1) comprises a transparent visor (2) to allow the user's vision once the air filtration device (1) has been placed on the user's head.

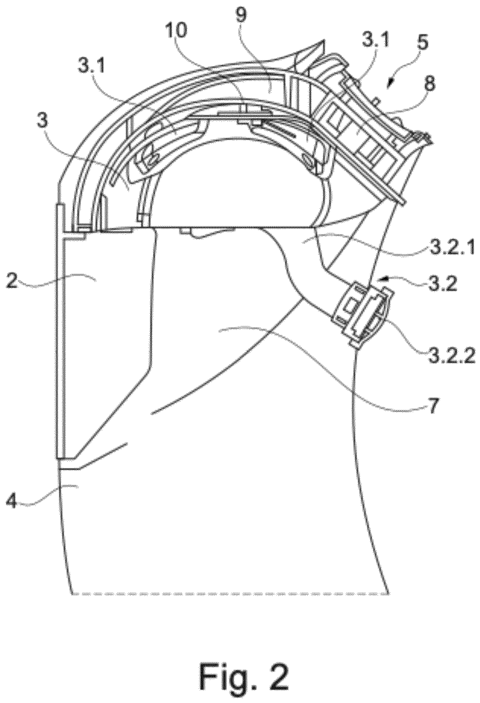

As can be seen in Figures 1 and 2, the visor (2) is attached to a holding structure (3). The fastening structure (3) is configured for fastening to the head of a user.

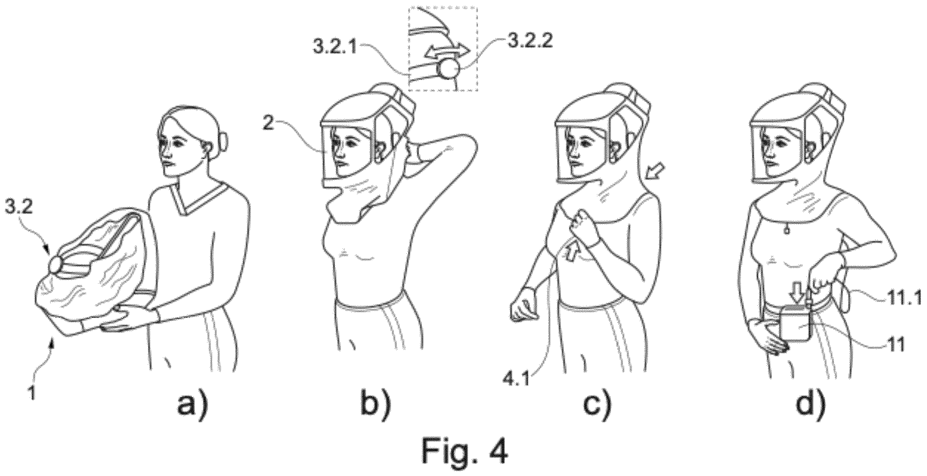

The visor (2) is arranged in such a way that, once the fastening structure (3) is attached to the user's head, the visor (2) is in front of the user's face and at a distance such that it leaves a space of adequate separation between the face and the visor (2), as shown in figures 4c and 4d. In this way, the possibilities of fogging of the visor (2) are limited and the discomfort that the visor (2) would cause if it were very close to the face is reduced.

The holding structure (3) comprises, as can be seen in Figure 2, protrusions (3.1) to support the user's head. It is contemplated that these protrusions (3.1) may be padded to improve user comfort.

Likewise, the fastening structure (3) comprises fastening means (3.2). In the example shown, the fastening means (3.2) comprise straps (3.2.1) and a hooking means (3.2.2). The attachment means (3.2.2) comprises means for adjusting the force of the attachment so that the user can properly attach the air filtration device (1) to the head.

The air filtering device (1) additionally comprises a hood (4) fixed both to the visor (2) and to the holding structure (3). When the support structure (3) is attached to the user's head, the hood (4) surrounds the head and neck of the user. user. Once the air filtration device (1) is attached to the user's head, the hood (4) and visor (2) define a closed chamber (7) around the user's head, as shown in figure 2.

Preferably, the hood (4) is made of a material that prevents the passage of air, such as a material in accordance with the UNE EN 13795 standard, which prevents the passage of infectious agents from outside the closed chamber (7) to the interior. and vice versa.

To ensure the tightness of the closed chamber (7), the hood (4) comprises hermetic closing means (4.1) (such as a cord, loop, etc.) in the lower part of the hood (4).

The lower part of the hood (4) is understood to be the section of the hood (4) that is at the lower end when the air filtration device (1) is attached to the user's head.

As can be seen in Figure 2, the air filtration device (1) comprises a control unit (10). In the example shown, the control unit (10) is fixed to the fastening structure (3).

In the example, an external unit (11) is also provided to supply electrical energy to the control unit (10). The external unit (11) comprises an electrical connection means (11.1) for the electrical connection of the external unit (11) to the control unit (10).

It should be noted that, although in this preferred embodiment the use of an external unit (11) for the supply of electrical energy is mentioned, it is not ruled out that the air filtration device (1) comprises an electric battery inside the unit. the closed chamber (7) for supplying electrical energy to the control unit (10).

Figure 4 shows the steps to follow to secure the air filtration device (1) to the user's head. These steps are: a) hold the air filtration device (1), so that the holding structure (3) is not covered by the hood (4), b) place the holding structure (3) on the head of the user so that the visor (2) is in front of the user's face and hook the straps (3.2.1) on the back of the head by means of the hooking means (3.2.2); c) make use of the closing means (4.1), in this case a cord, of the hood (4) ensuring the tightness of the closed chamber (7); and d) fix the external unit (11) to the waist, connecting the external unit (11) to the air filtration device (1) by means of the electrical connection means (11.1).

The air filtering device (1) includes an air inlet (5) to allow the passage of breathable air inside the closed chamber (7). Said air inlet (7) comprises, in turn, an inlet filter (5.1) for filtering the air that goes through said air inlet (5), so as to ensure that the air entering the closed chamber ( 5) is free of infectious agents.

In order to generate an air flow (12) from the outside of the air inlet (5) to the inside of the closed chamber (7), the air inlet (5) connects, on the one hand, to the outside of the closed chamber (7) and, on the other, a fan (8), more specifically a centrifugal fan (8).

The fan (8) is configured to suck the air from outside the closed chamber (7) through the inlet filter (5.1). Likewise, the fan (8) is configured to introduce a flow rate, for example, of more than 1201/min, into an air duct (9).

The air duct (9) is configured to distribute the flow of the air flow (12) to a front part of the closed chamber (7).

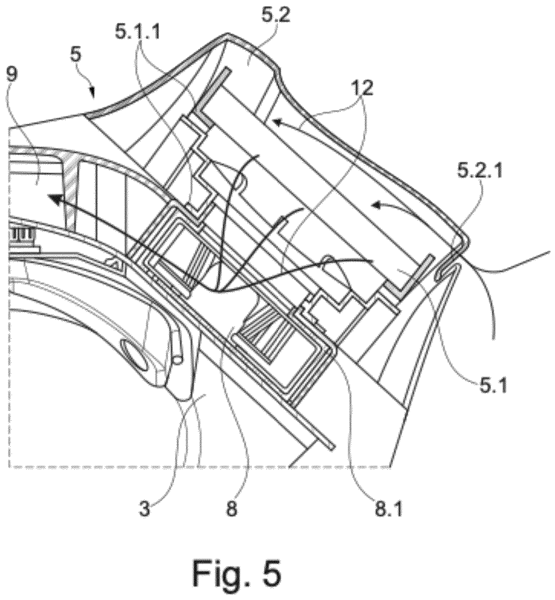

As can be seen in Figure 5, the air inlet (5) comprises a cover (5.2) that protects the inlet filter (5.1) from splashes or impacts that could affect the filtration capacity of the inlet filter (5.1). ). Furthermore, the cover (5.2) includes an inlet hole (5.2.1) through which outside air enters.

The inlet filter (5.1) is mounted inside an inlet frame (5.1.1). The inlet frame (5.1.1) can be fixed to a cavity (8.1) of the support structure (3) where the fan (8) is arranged, by means of a bayonet closure.

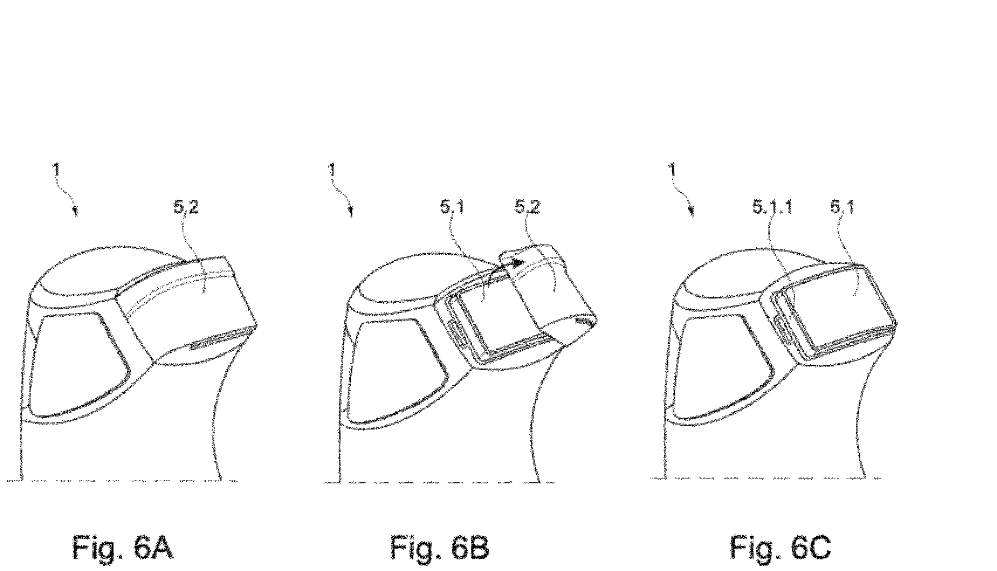

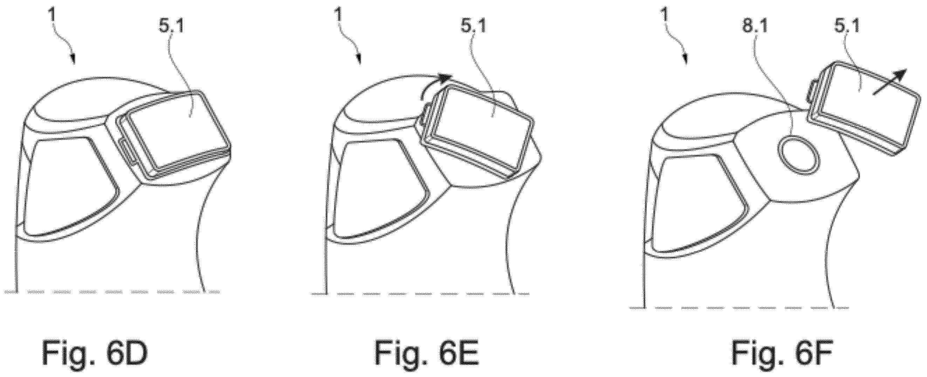

As can be seen in figure 5, the hood (4) remains fixed to the air inlet (5) by trapping between the inlet frame (5.1.1) and the cavity (8.1). As can be seen in Figures 6A to 6F, the air inlet (5) is configured to be able to extract the inlet filter (5.1). To do this, firstly, the cover (5.2) is uncoupled, as can be seen in figures 6A to 6C, so that the input frame (5.1.1) is uncovered. Next, and as can be seen in figures 6D to 6F, the inlet frame (5.1.1) is rotated, disengaging it from the fan (8) and separated from the air outlet (6). Finally, to place a new input filter (5.1) with its corresponding input frame (5.1.1), these steps are repeated in the opposite direction.

As can be seen in Figure 2, the air duct (9) is located at the top of the air filtration device (1).

As mentioned above, rear, top, side and front can be understood as the different positions of the device relative to the user's head when the air filtration device (1) is in its position of use.

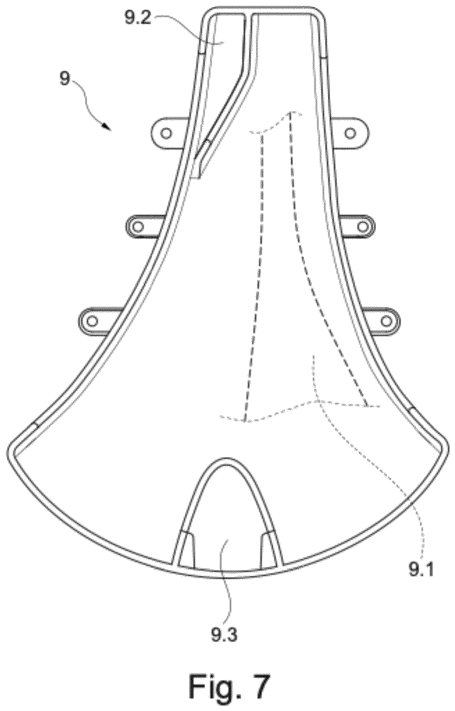

As can be seen in Figure 7, the air duct (9) comprises several subducts (9.1) which have been illustrated schematically, so that their location and extension can vary depending on the design and needs. The subducts (9.1) allow a homogeneous distribution of the air flow (12) at the outlet of the air duct (9), which is the section opposite the fan (8). The outlet of the air duct (9) is arranged in a front part of the closed chamber (7), more specifically in an upper part of the visor (2).

The upper part of the visor (2) is understood as the section of the visor (2) that is at the upper end when the air filtration device (1) is in its position of use.

It is also provided that the air duct (9) comprises an LED lighting means (9.3) in communication with the control unit (10). This LED lighting means (9.3) would be located in such a way that it is in the range of vision of the user when the air filtration device (1) is in its position of use.

In this way, the control unit (10) can inform the user of different states of the air filtration device (1) by means of a series of colors (for example, green if the state is optimal, orange if the external unit (11) is downloaded, etc.). Likewise, the air duct (9) comprises a housing (9.2) to house a sensor of air flow characteristics (12) that enters the air duct (9).

Preferably, the air flow characteristics sensor (12) is a flow meter, which will be in communication with the control unit (10), which receives information on the inlet flow rate of the air flow (12).

It is not ruled out that said air flow characteristics sensor (12) is a CO₂ concentration sensor, a pressure sensor, a temperature sensor, etc. Nor is it ruled out that the device includes several air flow characteristic sensor mists (12).

With the information obtained from the air flow characteristics sensor (12), the control unit (10) can control the fan (8) (turning it on, turning it off, varying the power and/or speed of rotation of the motor, etc.) so that a maximum pressure difference with respect to the outside atmospheric pressure of up to 0.5 mBar is maintained inside the closed chamber (7).

This pressure difference between the outside pressure (for example 1 atmosphere, that is, 1013.25 mBars) and the pressure inside the closed chamber (7) (in this example between 1013.25 mBars and 1013.75 mBars) allows that the user is adequately ventilated.

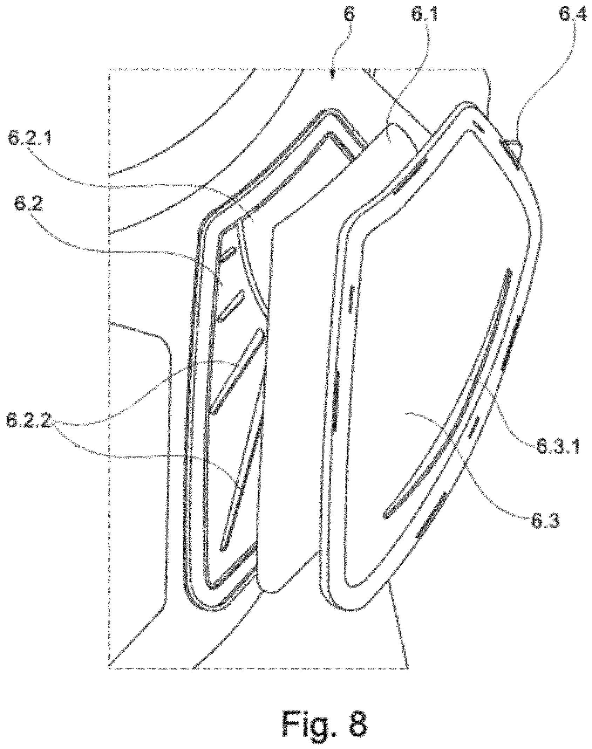

As can be seen in Figure 8, the air outlet (6) is located on at least one side of the air filtration device (1) and includes an outlet filter (6.1). This allows the user's infectious agents to be effectively filtered by the outlet filter (6.1) upon exiting the closed chamber (7).

To ensure that the pressure difference in the closed chamber (7) is less than 0.5 mBar, the inlet filter (5.1) and the outlet filter (6.1) are sized depending on the inlet flow rate of the air flow. (12). That is, a higher flow rate requires a larger surface area of the inlet filter (5.1) and the outlet filter (6.1).

In the non-limiting example shown in the drawings, the air filtration device (1) comprises two air outlets (6). The air outlets (6) can be the same or have different characteristics. Each air outlet (6) comprises an outlet filter (6.1) and they are arranged on each side of the device (1). In this way, the effective surface area for the outlet filter (6.1) is doubled and a better distribution of the air flow (12) is obtained through the visor (2) and the user's face.

As shown in Figure 8, each air outlet (6) comprises a side frame (6.2) fixed to the hood (4). Said side frame (6.2) comprises an outlet hole (6.2.1) for the release of air from inside the closed chamber (7).

Likewise, the side frame (6.2) comprises ribs (6.2.2) on an outer surface of the side frame (6.2). The ribs (6.2.2) allow the condensation of humidity from the air that passes through the air outlet (6) to be absorbed. In addition, the ribs (6.2.2) allow a space to be left between the outlet filter (6.1) and the outer surface of the side frame (6.2), thereby achieving that most of the surface of the outlet filter (6.1) ) can be used to filter the air that passes through the air outlet (6).

In the non-limiting example shown in the drawings, a side cover (6.3) is attached to the side frame (6.2) by elastic snap or clipping. The side cover (6.3) protects the outlet filter (6.1) from splashes or impacts that could affect the filtration capacity of the outlet filter (6.1). The side cover (6.3) comprises an opening (6.3.1) for the passage of the air flow (12) through the air outlet (6).

Both the side frame (6.2) and the side cover (6.3) have a tab (6.4) to facilitate the detachment of the side cover (6.3) when the outlet filter (6.1) has to be replaced.

Likewise, the side cover (6.3) has a curved surface that defines an air chamber between the surface of the outlet filter (6.1) and the side cover (6.3). In this way, it is ensured that the entire surface of the outlet filter (6.1) can be used for filtering the air that passes through the air outlet (6).

The side frame (6.2) comprises ribs (6.2.2) on the outer surface of the side frame (6.2). Said ribs (6.2.2) allow the condensation of humidity from the air that passes through the air outlet (6) to be absorbed.

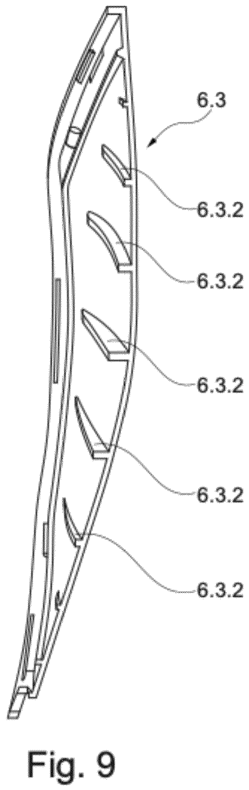

Additionally, the side cover (6.3) includes ribs (6.3.2) on the curved surface, as as can be seen in figure 9.

In an example of a preferred embodiment, the outlet filter (6.1) is first arranged on the ribs (6.3.2) of the side cover (6.3), to then fix the side cover (6.3) to the side frame (6.2) without touching the outlet filter (6.1).

In another example of a preferred embodiment, the outlet filter (6.1) is first arranged on the ribs (6.2.2) of the side frame (6.2), to then fix the side cover (6.3) to the side frame (6.2) without touching the outlet filter (6.).

The inlet filter material (5.1) is expected to have a filtering capacity according to category P3 according to the UNE EN 143:2021 regulation, which specifies the operating requirements and test methods of filters designed to filter particles ( that is, capable of filtering, among other things, viruses).

The air introduced inside the closed chamber (7) receives high filtration. This is very important in case the user is in contact with patients with highly contagious diseases.

Likewise, it is expected that the output filter material (6.1) has a filtering capacity in accordance with a type II category according to the UNE EN 14683:2019 regulation, which specifies the operating requirements and test methods of filters designed for filter bacteria.

In this way, the inlet filter (5.1) will filter all the air that enters the closed chamber (7), protecting the user against particles and viruses from the outside, and the exhaled air will be filtered by the outlet filter (6.1), filtering bacteria, that is, filtering the contribution that the user can make to the filtered air in the air inlet (5).

The air expelled through the air outlet (6) has been previously filtered by the inlet filter (5.1). This air only contains the user's infectious agents, so it is not necessary to use an outlet filter (6.1) with a filtering capacity according to the UNE EN 143:2021 standard.

The inlet filter material (5.1) has a higher filtering capacity than the intake filter. output (6.1). In this way, the necessary surface of the outlet filter (6.1) is reduced for the correct operation of the air filtering device (1).

For example, in the event that the material of the outlet filter (6.1) were in accordance with the UNE EN 143:2021 regulations (category P3) and the surface of the outlet filter (6.1) is not modified, a difference would be required. pressure between the outside and inside of the closed chamber (7) greater than 0.5 mBars, which would cause inconvenience to the user. This would require increasing the surface area of the outlet filter (6.1), and therefore of the air outlet (6) and the air filtration device (1), which would result in inconvenience for the user (for example, due to the increase weight, volume, etc.).

It is also contemplated that the external unit (11) comprises speakers so that the user's voice can be clearly heard outside the closed chamber (7).

Likewise, the possibility is contemplated that the control unit (10) is arranged in the external unit (11).

It is also envisaged that the control unit (10) can be configured to perform the following tasks. fan noise cancellation (8), thereby improving the user's hearing; Bluetooth^{®} communication for communication by telephone, use of a digital background, or for communication with a user's headset; increase the volume of the speaker of the external unit (11) for those patients with hearing problems; analyze the frequency of use of the air filtration device (1), the user's movements; verify the user's identity; inform the user when it is necessary to replace the input filter and/or the output filter (6.1), normally by means of the LED lighting means (9.3).

## Claims

1. Air filtration device (1) for protection that comprises:
- a fastening structure (3) configured to be fastened to the head of a user;
- a visor (2) associated with the support structure (3);
- a hood (4) associated with the fastening structure (3), said hood (4) defining a closed chamber (7) after fastening the fastening structure (3) to the user's head;
- a rear air inlet (5) comprising an inlet filter (5.1);
- an air outlet (6); and
- a fan (8) configured to generate an air flow (12) from the air inlet (5) through an upper air duct (9) and through a front part of the closed chamber (7) to the outlet. air (6);
**characterized in that** the air outlet (6) is arranged on at least one side and comprises an outlet filter (6.1), a side frame (6.2) and a side cover (6.3), the side cover (6.3) presenting by at least one opening (6.3.1) for the passage of air and a curved surface that defines an air chamber between the outlet filter (6.1) and the side cover (6.3), the side cover (6.3) being configured to be fixed to the side frame (6.2), so that the outlet filter (6.1) is fixed between the side frame (6.2) and the side cover (6.3).

2. Air filtration device (1), according to claim 1, **characterized in that** the side frame (6.2) comprises an outer surface that is provided with ribs (6.2.2) on which the outlet filter (6.1).

3. Air filtering device (1), according to any of the preceding claims, **characterized in that** the side cover (6.3) comprises ribs (6.3.2) on the curved surface where the outlet filter (6.1) is located.

4. Air filtration device (1), according to any of the preceding claims, **characterized in that** the upper air duct (9) comprises a plurality of subducts (9.1) for a homogeneous distribution of the air flow (12) throughout. length of the visor (2).

5. Air filtering device (1), according to any of the preceding claims, **characterized in that** the inlet filter (5.1) has a greater filtering capacity than the outlet filter (6.1).

6. Air filtration device (1), according to any of the previous claims, **characterized in that** it comprises an external electrical power unit (11) that can be fixed to the user's waist.

7. Air filtration device (1), according to any of the preceding claims, **characterized in that** it comprises sound amplification means that at least comprise a microphone, a headset and/or a speaker to allow communication. between the user and the outside.

8. Air filtration device (1), according to claim 7, **characterized in that** the sound amplification means are configured to cancel the sound emitted by the air flow (12) and/or the ambient sound of the abroad.

9. Air filtration device (1), according to any of the preceding claims, **characterized in that** it comprises a control unit (10) configured to regulate the speed of the fan (8).

10. Air filtration device (1), according to claim 9, **characterized in that** it comprises at least one sensor of air flow characteristics (12) inside the closed chamber (7), the unit being control unit (10) configured to receive information from the airflow characteristics sensor (12), in particular, to continuously monitor the information received from the airflow characteristics sensor (12).

11. Air filtration device (1), according to any of claims 9 to 10, **characterized in that** the control unit (10) is configured to maintain a maximum pressure difference with respect to the outside atmospheric pressure at inside the closed chamber (7) up to 0.5 mBar.

12. Air filtration device (1), according to any of the claims 9 to 11, **characterized in that** it comprises identification means of the device (1) of univocally by means of a unique digital label processed by the control unit (10).

13. Air filtration device (1), according to any of claims 9 to 12, **characterized in that** it comprises data encryption means processed by the control unit (10), in particular, by means of a cryptographic algorithm, more particularly, the encryption being carried out through "blockchain" and/or "tokenization" technology.

14. Air filtration device (1), according to any of claims 9 to 13, **characterized in that** the control unit (10) comprises wireless internet connection means, in particular via WIFI, Bluetooth^{®}, Zigbee, NFC, GSM, LoRa and/or Sigfox, directly to a server or to an intermediate node, more particularly, using an ISM band frequency and a proprietary protocol.

15. Cleaning system for an air filtration device (1) according to any of the preceding claims, **characterized in that** it comprises means for sterilizing the device (1) by means of IIV-C radiation.
